# EUROPEAN PATENT SPECIFICATION

(11) **EP 2 090 226 B1**
(45) Date of publication and mention of the grant of the patent: **17.08.2011**
(21) Application number: 07702317.4
(22) Date of filing: 08.02.2007
(51) Int. Cl.: A61B 5/1455

(54) **FINGER CLIP TYPE BLOOD OXYGEN SATURATION MEASURING APPARATUS**
GERÄT ZUR MESSUNG DER BLUTSAUERSTOFF-SÄTTIGUNG VOM FINGERCLIP-TYP
APPAREIL DE MESURE DE SATURATION DU SANG EN OXYGÈNE DE TYPE PINCE DIGITALE

(30) Priority: 27.11.2006 CN 200610144965
(43) Date of publication of application: 19.08.2009
(73) Proprietor: Beijing Choice Electronic Technology Co., Ltd., Beijing 100039 (CN)
(72) Inventor: WANG, Weihu, Beijing 100039 (CN); WU, Peng, Beijing 100039 (CN); CHEN, Lei, Beijing 100039 (CN)
(74) Representative: Mittler, Enrico
(86) International application number: PCT/CN2007/000448
(87) International publication number: WO 2008/064534

(56) References cited:
- EP-A1- 1 547 517
- WO-A2-01/73394
- CN-A- 1 418 072
- CN-A- 1 798 520
- CN-C- 1 098 053
- US-A- 3 152 587
- US-A- 3 810 460
- US-A- 5 490 523

## Description

### Technical Field

The present invention relates to a fingertip blood oxygen saturation measuring apparatus, in particular, to a fingertip oximeter which can ensure the measuring accuracy.

The term "photoelectric cell" appearing in this specification means all photoelectric sensing elements for measuring the blood oxygen saturation, which can send signals on one side of a measured fingernail and receive the signals on the other side of the fingernail.

### Background of the Invention

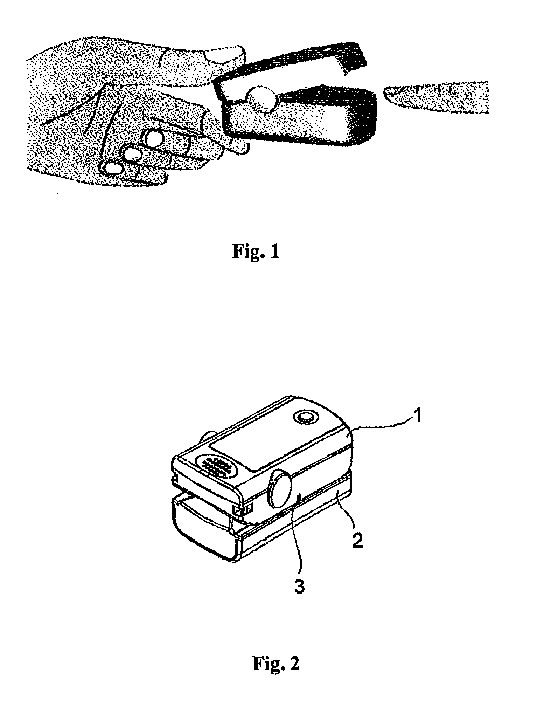

At present, the fingertip pulse oximeter available in the market is shown in Figure 1. The apparatus is of small volume, convenient carrying, and simple use, and is extremely welcomed by people. However, the apparatus has also some drawbacks in use. Another apparatus is described in US3,152,587, disclosing the features according to the preamble of claim 1.

For example, an inaccurate displaying or instable displaying of the measured data may occur sometimes during the measurement. The reason is that the measured person's finger positioning in the finger hole is not accurate enough during the measurement. Furthermore, as the thickness and length of the fingers of different measured persons are different, the place of the fingernail may not aim at the photoelectric sensing element in the fingertip pulse oximeter. Because the photoelectric cell in the fingertip pulse oximeter is out of sight from the exterior, the measured person cannot further attempt to aim at the photoelectric cell after the finger is clamped to be measured, which leads to the above mentioned phenomena of inaccurate positioning of the finger.

### Summary of the Invention

The object of the present invention is to provide a fingertip blood oxygen saturation measuring apparatus, which can accurately measure the blood oxygen saturation in a simple way by setting an exterior mark.

For this purpose, according to one aspect of the present invention, a blood oxygen saturation measuring apparatus is provided, which comprises an upper shell, a lower shell, and an elastic restoring structure, characterized in that a mark is arranged on a lateral side surface of the upper and/or the lower shell, and the mark is provided to be in alignment with the vertical position of a photoelectric sensing chip which is arranged inside the shell. A fluorescence mark is used as the mark in the convex or concave manner.

Preferably, the mark is arranged on the lateral side surface of the shell in a convex or concave manner.

Preferably, a fluorescence mark is used as the mark.

Preferably, the present invention further comprises a detection sensing chip, an internal power source, an internal photoelectric sensing chip, a detecting, processing and controlling circuit, and a display screen.

According to another aspect of the present invention, a blood oxygen saturation measuring probe apparatus is provided, which is connected with a portable oximeter or a monitoring instrument by cables or socket connectors, the oximeter comprises an upper shell, a lower shell, and an elastic restoring structure, characterized in that a mark is provided on a lateral side surface of the upper and/or the lower shell, and the mark is in alignment with the vertical position of a detection photoelectric sensing chip which is arranged inside the shell. A fluorescence mark is used as the mark.

Preferably, the mark is arranged on the lateral side surface of the shell in a convex or concave manner.

Preferably, the fluorescence mark is used as the mark in the convex or concave manner.

According to the present invention, the humanized mark is adopted to observe the measured position of the measured person's finger. Even if in the case of weak light environment or a lazy eye person, the accurate and reliable blood oxygen saturation measured data can be acquired.

### Brief Description of the Drawings

Figure 1 is a schematic view of usage of a fingertip pulse oximeter according to the prior art;
Figure 2 is a perspective view of a fingertip blood oxygen saturation measuring apparatus according to the present invention;
Figure 3 is a back view of the fingertip blood oxygen saturation measuring apparatus according to the present invention; and
Figure 4 is a schematic view of a separated blood oxygen saturation measuring probe apparatus according to the present invention.

### Detailed Description of the Preferred Embodiments

Next, the embodiments of the present invention are described in more detail in combination with accompanying figures.

As shown in Figure 2, according to an embodiment of the fingertip blood oxygen saturation measuring apparatus according to the present invention, the fingertip blood oxygen saturation measuring apparatus comprises an upper shell 1, a lower shell 2, an elastic restoring structure, a detection photoelectric sensing chip, an internal power source, an internal photoelectric sensing chip, a detecting, processing and controlling circuit, and a display screen. A mark 3 is arranged on a lateral side surface of the upper shell 1. The vertical line as the mark 3 on the lateral side surface of the upper shell 1 is in alignment with the vertical position of the detection photoelectric sensing chip arranged inside the upper shell 1. The mark 3 is arranged on the lateral side surface of the upper shell 1 in a convex manner. The mark 3 is also represented its position by fluorescence.

A photoelectric cell A-OE-06 or A-OE-02 can be used as the detection photoelectric sensing chip.

According to the present invention, when the measurement is conducted, the measured person extends his/her one finger into between the upper shell 1 and the lower shell 2. Before the upper shell 1 and the lower shell 2 are loosened from each other, the mark 3 which is arranged on the lateral side surface of the upper shell 1 is made to aim at the fingernail root position of the measured finger. The mark 3 which is arranged on the lateral side surface of the loosened upper shell 1 is rightly aimed at the fingernail root position of the measured finger. At the moment, if the finger positioning has a certain deviation, the finger can be adjusted according to the position of the mark 3 which is arranged on the lateral side surface of the upper shell 1. The accurate measurement can be conducted until the finger positioning is accurate.

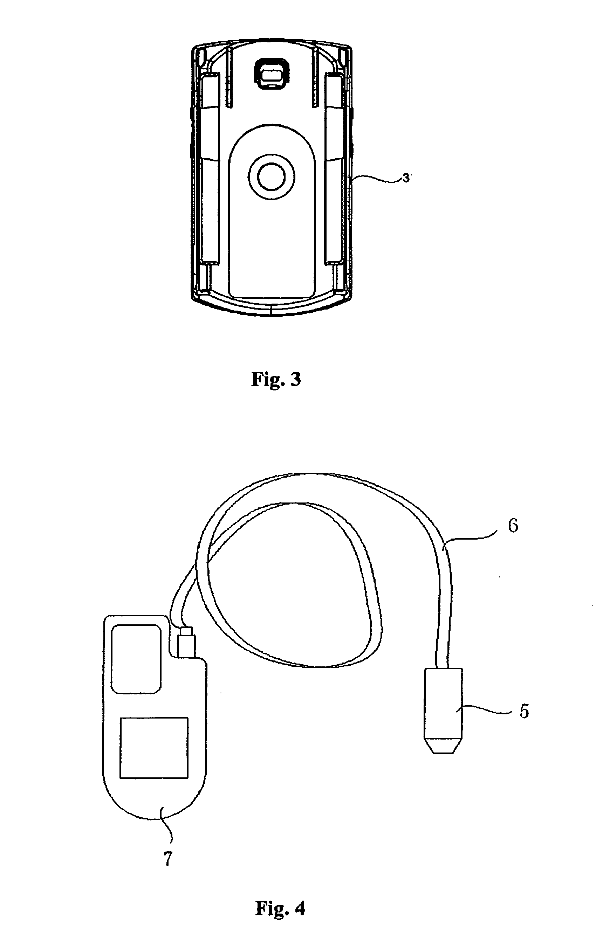

As shown in Figure 4, according to another embodiment of the present invention, the blood oxygen saturation measuring probe apparatus 5 is connected to a prior art portable blood oxygen instrument or a monitoring instrument 7 by cables 6 or socket connectors. With respect to the first embodiment, the main difference of the blood oxygen saturation measuring probe apparatus 5 of the present invention is that the internal power source, the detecting, processing and controlling circuit, as well as the display screen, etc. are arranged inside the portable blood oxygen instrument or the monitoring instrument 7.

The blood oxygen saturation measuring probe apparatus 5 according to the present invention comprises an upper shell 1, a lower shell 2, an elastic restoring structure, and a detection photoelectric sensing chip. A mark 3 is arranged on the lateral side surface of the upper shell 1. The vertical position of the mark 3 on the lateral side surface of the upper shell 1 is provided to be in alignment with the vertical position of the detection photoelectric sensing chip arranged inside the upper shell 1. The mark 3 is arranged on the lateral side surface of the upper shell 1 in a convex manner. The position of the mark 3 is also represented by fluorescence.

According to another embodiment of the present invention, the mark 3 can also be arranged on the lateral side surface position of the lower shell 2.

According to another embodiment of the present invention, the mark 3 can also be arranged on both the lateral side surface of the upper shell 1 and the lateral side surface of the lower shell 2.

When the measurement is started, the measured person extends his/her one finger into the measuring probe apparatus 5. The mark 3 arranged on the lateral side surface is made to be aimed at the fingernail root position of the measured finger, so as to adjust the position of the finger, and the accurate measurement can be conducted until the finger positioning is accurate.

According to the different demands of the measured persons, the mark 3 which is arranged on the lateral side surface may be convex or concave. The position of the mark 3 is represented by fluorescence, which satisfies the usage in the case of somewhat dark environment or lazy eye users. Whether the finger positioning is accurate or not can be indicated by fluorescence, or by touching the mark by the other hand, so as to ensure the accurate measurement.

The present invention is described above in detail according to a plurality of embodiments. However, those skilled in the art should understand that various modifications and improvements can be made on the present invention. The scope of protection will be defined by the attached claims of the present invention.

## Claims

1. A blood oxygen saturation measuring apparatus comprising an upper shell (1), a lower shell (2) and an elastic restoring structure, wherein a mark (3) is arranged on a lateral side surface of the upper and/or the lower shell (1, 2), and the mark (3) is provided to be in alignment with a vertical position of a detection photoelectric sensing chip which is arranged inside the shell (1, 2), **characterized in that** the mark (3) is a fluorescence mark.

2. The blood oxygen saturation measuring apparatus according to claim 1, **characterized in that** the mark (3) is arranged on the lateral side surface of the shell (1, 2) in a convex or concave manner.

3. The blood oxygen saturation measuring apparatus according to claim 1, **characterized by** further comprising an internal power source, an internal photoelectric sensing chip, a detecting, processing and controlling circuit, and a display screen.

## Patentansprüche

1. Gerät zur Messung der Blutsauerstoffsättigung, umfassend einen oberen Clipschenkel (1), einen unteren Clipschenkel (2) und eine elastische Rückholstruktur, wobei eine Marke (3) auf einer Seitenfläche des oberen und/oder des unteren Clipschenkels (1, 2) angeordnet ist und die Marke (3) sich in Ausrichtung mit einer vertikalen Position eines photoelektrischen Detektionssensorchips, welcher im Inneren der Clipschenkel (1,2) angeordnet ist, befindet,
**dadurch gekennzeichnet,**
**dass** die Marke (3) eine Fluoreszenzmarke ist.

2. Gerät nach Anspruch 1, **dadurch gekennzeichnet, dass** die Marke auf der Seitenfläche des Clips in konvexer oder konkaver Weise angeordnet ist.

3. Gerät nach Anspruch 1, darüberhinaus **gekennzeichnet durch** eine interne Leistungsquelle, einen internen photoelektrischen Sensorchip, einen Detektions-, Prozess- und Steuerschaltkreis und einen Displayschirm.

## Revendications

1. Appareil de mesure de la saturation du sang en oxygène comprenant une coque supérieure (1), une coque inférieure (2) et une structure de rétablissement élastique, dans lequel un repère (3) est disposé sur une surface latérale de la coque supérieure et/ou de la coque inférieure (1, 2) et le repère (3) est prévu de sorte à être en alignement avec une position verticale d'une puce de détection à captage photoélectrique qui est disposée à l'intérieur de la coque (1, 2), **caractérisé en ce que** le repère (3) est un repère fluorescent.

2. Appareil de mesure de la saturation du sang en oxygène selon la revendication 1, **caractérisé en ce que** le repère (3) est disposé sur la surface latérale de la coque (1, 2) d'une manière convexe ou concave.

3. Appareil de mesure de la saturation du sang en oxygène selon la revendication 1, **caractérisé en ce qu'**il comprend en outre une source d'alimentation interne, une puce à captage photoélectrique interne, un circuit de détection, de traitement et de contrôle et un écran d'affichage.
